# EUROPÄISCHE PATENTSCHRIFT

(11) **EP 0 681 834 B1**
(45) Veröffentlichungstag und Bekanntmachung des Hinweises auf die Patenterteilung: **08.12.1999**
(21) Anmeldenummer: 94107025.2
(22) Anmeldetag: 05.05.1994
(51) Int. Cl.: A61K 9/50, A61K 9/16

(54) **Mikrokapsel sowie Verfahren und Vorrichtung zu ihrer Herstellung**
Microcapsule as well as a method and a device to produce it
Microcapsule, ainsi qu'un procédé et un appareil pour la produire

(43) Veröffentlichungstag der Anmeldung: 15.11.1995
(73) Patentinhaber: SCHREZENMEIR, Jürgen, D-55128 Mainz (DE); Pommersheim, Rainer, 76185 Karlsruhe (DE); Vogt, Walter, Dr., D-65207 Wiesbaden (DE)
(72) Erfinder: SCHREZENMEIR, Jürgen, D-55128 Mainz (DE); Pommersheim, Rainer, 76185 Karlsruhe (DE); Vogt, Walter, Dr., D-65207 Wiesbaden (DE)
(74) Vertreter: Pöhner, Wilfried Anton, Dr.

(56) Entgegenhaltungen:
- WO-A-92/06678

## Beschreibung

Die Erfindung bezieht sich auf eine Mikrokapsel, insbesondere zum Einsetzen in Gewebe von Lebewesen oder für biotechnologische Anwendungen, mit einem lebende Zellen und/oder Enzyme enthaltenden, vorzugsweise kugelförmigen Kern und einer ihn umschließenden Hülle, wobei die Hülle in radialer Richtung aus mehreren übereinander angeordneten Einzelschichten aufgebaut ist und die Einzelschichten den Kern allseitig vollständig umschließen.

Um die Funktion in das Gewebe von Lebewesen eingesetzter Zellen langfristig aufrechtzuerhalten, ist eine die Zellen enthaltende Mikrokapsel vollständig einschließende Hülle erforderlich. Die Aufgabe dieser Hülle besteht beispielsweise darin, die Mikrokapsel mechanisch stabil zu gestalten, um das Verbleiben der Zellen am gewünschten Ort sicherzustellen. Weiterhin besteht die Aufgabe darin, die Versorgung der Mikrokapsel von außen zu ermöglichen, Abwehrreaktionen des Lebewesens und der eingeschlossenen Zellen zu vermeiden und trotzdem den Transport der von den eingesetzten Zellen produzierten Substanzen von innen nach außen zu ermöglichen. Beispielsweise eignet sich eine Mikrokapsel mit den beschriebenen Eigenschaften zum Einsetzen Langerhans'scher Inseln in das Gewebe von Diabetikern.

Ähnliche Anforderungen werden an Mikrokapseln für biotechnologische Anwendungen gestellt. Auch hier ist beabsichtigt, die eingeschlossenen Zellen oder Enzyme langfristig am Leben und in optimal aktivem Zustand zu erhalten und einen kontrollierten Stoffaustausch zu gewährleisten. Der Einschluß in eine stabile Kapsel schützt einerseits den Kern vor schädigenden Einwirkungen von außen, z.B. vor Infektionen, und andererseits wird eine Kontamination des umgebenden Mediums durch die Zellen in der Kapsel vermieden, was besonders bei Nutzung gentechnologisch manipulierter Organismen zusätzliche Sicherheit gewährleistet. Die, im Vergleich zu den eingeschlossenen Zellen und Enzymen, sehr großen Abmessungen der Kapseln bedingen darüber hinaus eine wesentliche Erleichterung in der Handhabung, besonders bei der Aufarbeitung und Reingewinnung der mit ihrer Hilfe erzeugten Substanzen. Auch ganz neue Prozesse werden möglich, beispielsweise die direkte Extraktion von Produkten schon im Bioreaktor durch Zusatz von organischen Lösungsmitteln, die nicht durch die einschließende Hülle geschützte Zellen in kurzer Zeit abtöten würden.

F. Lim und A. Sun (Zeitschrift "Science" Band 210, Seiten 908-910, Jahrgang 1980) benutzten eine aus Alginat und Polylysin aufgebaute Schicht, um Langerhans'sche Inseln einzukapseln. Das von Lim und Sun vorgeschlagene Verfahren sieht die Anbringung nur einer einzigen Schicht um den Kern vor.

Ein elektrostatischer Tröpfchen-Generator wurde von M. Hommel et al. (EP 0 167 690) vorgeschlagen. Dieser Generator produziert von Polyelektrolyten umschlossene Kapseln nach dem Prinzip von Lim und Sun. Auch diese Kapseln sind nur von einer einzigen, während eines einzelnen Prozeßschrittes erzeugten Schicht umschlossen.

Aus der GB 2 009 698 A ebenso wie aus der WO 92/06678 sind Kapseln mit mehrschichtigem Aufbau bekannt, deren Hüllen aus speziellen Substanzen aufgebaut sind.

Es ist sehr problematisch, in einem einzigen Prozeßschritt eine Schicht zu erhalten, deren Eigenschaften auf alle praktischen Anforderungen abgestimmt sind. Daher ist es mit den konventionellen Methoden nicht möglich, beispielsweise eine Kapsel mit einer eine langfristige Funktion der implantierten Zellen ermöglichenden Schicht zu erhalten.

Hiervon ausgehend liegt der Erfindung das Problem zugrunde, Mikrokapseln herzustellen, deren sie umschließende Hülle hinsichtlich verschiedener Eigenschaften optimiert ist.

Erfindungsgemäß wird diese Aufgabe dadurch gelöst, daß die Einzelschicht aus einem Netzwerk miteinander verflochtener Makromoleküle besteht, die eine poröse Membran bilden, daß die Poren der Einzelschichten in radialer Richtung zur Bildung eines Durchlasses miteinander in Verbindung stehen, daß die benachbarten Einzelschichten kovalent und/oder elektrostatisch aneinander gebunden sind, daß mindestens eine der Einzelschichten mechanisch stabil aufgebaut ist und daß mindestens eine der Einzelschichten eine Maschen weite aufweist, die größer oder gleich dem Durchmesser der größten, zur Versorgung der im Kern enthaltenen Zellen benötigten und/oder der den Enzymen zugeführten und/oder von ihnen produzierten Moleküle ist.

Der Kerngedanke der Erfindung besteht darin, mehrere jeweils hinsichtlich einer Eigenschaft optimierte Einzelschichten in mehreren Prozeßschritten in radialer Richtung übereinander um den Kern herum anzubringen. Alle Einzelschichten umschließen den Kern vollständig. Eine solche Einzelschicht besteht aus einem räumlichen Netzwerk von miteinander verflochtenen Makromolekülen, die in ihrer Gesamtheit eine Membran von poröser Struktur bilden, ähnlich einem Schwammtuch oder einem Filtervlies. Sie entsteht durch Adsorption der einzelnen Makromoleküle aus einer verdünnten Lösung des jeweiligen Polymeren an der Oberfläche der im Aufbau befindlichen Kapsel, beginnend mit einem Partikelchen aus dem nackten Kernmaterial. Die Maschenweite (bzw. Porengröße) und die mechanische Stabilität werden dabei durch die Art des jeweiligen Polymeren, die Abscheidungsbedingungen und die Art der vorhergehenden Schicht bestimmt. Die Poren stehen miteinander in Verbindung (sind offen) und mit dem jeweiligen Lösungsmittel, vorzugsweise Wasser, gefüllt, d.h. die Schicht ist gequollen. Der Stoffaustausch erfolgt durch Diffusion der gelösten Stoffe in diesem Quellungswasser. Nur Moleküle, die kleiner oder gleich der Maschengröße sind, können die Schicht passieren. Die Bindung der Einzelschichten untereinander, also in radialer Richtung, erfolgt durch eine kovalente Bindung der Makromoleküle oder, falls Ionen darin enthalten sind, elektrostatisch. Von diesen Einzelschichten ist erfindungsgemäß mindestens eine so aufgebaut, daß sie eine ausreichende mechanische Stabilität aufweist, wodurch der gesamten Mikrokapsel ebenfalls eine entsprechend hohe Festigkeit verliehen wird, um sie ausreichend stabil für eine Verpflanzung oder eine technologische Anwendung zu gestalten. Eine andere Schicht ist durch Vernetzen von Molekülen bezüglich ihrer Maschenweite so gestaltet, daß sie dem Durchmesser der größten vom Kern zur Versorgung benötigten oder von den Enzymen umgesetzten Moleküle entspricht. Größere Moleküle, wie z.B Antikörper, die mit dem Kern unerwünschte Abwehrreaktionen eingehen würden, können diese Schicht nicht durchdringen, hingegen können Nährstoffe und andere erwünschte Moleküle passieren. Die Herstellung der Mikrokapseln erfolgt in mehreren Prozeßschritten, in denen die einzelnen Schichten sukzessive übereinander um den Kern herum angebracht werden.

Die mit der Erfindung erzielten Vorteile bestehen vornehmlich darin, daß die sich zu einem problemlosen Einpflanzen in Gewebe von Lebewesen und für biotechnologische Anwendungen eignende Mikrokapsel allen praktischen Anforderungen genügt. Dadurch ist möglich, Enzyme, Hormone oder andere Stoffe, die der Körper beispielsweise durch einen krankheitsbedingten Ausfall der Produktionszellen, nicht in dem nötigen Maße herzustellen in der Lage ist, von der Kapsel liefern zu lassen, die Kapsel durch den Körper des Lebewesens zu ernähren und dadurch eine langfristige, sichere Funktion zu gewährleisten. In prinzipiell gleicher Weise lassen sich die Kapseln in einem Reaktor, der den Überlebensnotwendigkeiten der eingeschlossenen Zellen bzw. der Wirksamkeit der Enzyme angepaßt ist, dazu verwenden, von außen zugeführte Stoffe durch die Wirkung der im Kern enthaltenen lebenden Zellen oder Enzyme in andere erwünschte Wertprodukte umzuwandeln.

Bevorzugt ist, die innere Schicht mit dem Kern biologisch verträglich zu gestalten, um sicherzustellen, daß er für längere Zeit die benötigten Enzyme, Hormone oder anderen Stoffe abgeben kann und daß hier keine unerwünschte Abwehrreaktion stattfindet.

Falls die Kapsel in das Gewebe des Lebewesens eingepflanzt werden soll, ist es zweckmäßig, die äußere Schicht mit ihrer Umgebung, also dem Gewebe verträglich zu gestalten, um Abwehrreaktionen des Lebewesens und eine Abstoßung der Kapsel zu vermeiden. Die äußere Schicht bestimmt die Verträglichkeit der gesamten Kapsel, da nur sie mit dem Gewebe in Berührung kommt. Befindet sich die Kapsel in einem Reaktor, ist ebenfalls eine Verträglichkeit der äußeren Einzelschicht mit ihrer Umgebung zur Sicherung der Funktionsfähigkeit erforderlich.

In der Realisation der Einzelschichten bestehen im Rahmen der Erfindung verschiedene Möglichkeiten. Eine vorteilhafte Ausgestaltung der Erfindung besteht darin, daß die Schichten aus einem Stoff mit ionischer und/oder kovalenter chemischer Bindung aufgebaut sind. Diese beiden Bindungstypen ermöglichen stabile Schichten, die unter Verwendung von insbesondere biologisch verträglichen, organischen Materialien aufbaubar sind.

Insbesondere bietet sich an, eine Einzelschicht aus einem Polymer aufzubauen, das biologisch gut verträglich und außerdem leicht aufbringbar ist. Bei Vernetzung von Polymeren sind sehr dünne Schichten auf chemischen Weg in einem biologisch sinnvollen Temperaturbereich in einer Flüssigkeit erzielbar. Außerdem entsteht eine stabile, in den mechanischen Eigenschaften und im Maschendurchmesser einstellbare Schicht.

Bereits bekannte und sehr gut geeignete, leicht zu verarbeitende Vertreter der Polymere sind Kombinationen von Polyanionen, Polykationen und neutralen Polymeren.

Hierzu bietet sich insbesonders als Polyanion Polyacrylsäure und/oder Polymethacrylsäure und/oder Polyvinylsulfonsäure oder Polyvinylphosphonsäure und/oder Schwefelsäureester polymerer Kohlenhydrate an.

Das Polykation ist zweckmäßigerweise Polyethylenimin und/oder Polydimethyldiallylammonium und/oder Chitosan.

Je nach der Säurestärke des umgebenden Mediums liegen Polyacrylsäure, Polymethacrylsäure, Polyethylenimin und Chitosan als Polyionen oder als mehr oder weniger elektrisch neutrale Polymere vor.

Die Eigenschaften der genannten Polyanionen und Polykationen sind bereits durch viele Versuche ermittelt worden und eignen sich demzufolge besonders zum Einsetzen in Gewebe von Lebewesen.

Ein Verfahren zur Herstellung der erfindungsgemäßen Mikrokapseln verläuft in der Weise, daß kleine Partikel, vorzugsweise Kügelchen, aus dem Kernmaterial, die einen Durchmesser im Bereich von O,3 bis 3 mm haben, in einem beweglichen feinmaschigen Korb, vorzugsweise aus Edelstahl, weiterverarbeitet werden. Um ein Verkleben der Kügelchen untereinander und mit der Gefäßwand zu verhindern, bietet sich während der nachfolgenden Prozess-Schritte ein kontinuierliches Umrühren und vertikales Schütteln an. Das Umrühren erfolgt in konventioneller Weise mit einem Rührwerk, während das vertikale Schütteln z.B. durch eine elektromotorisch bewegte Aufhängung bewirkt wird.

Der Korb mit den Kügelchen wird nacheinander mit Wasser und/oder geeigneten Salzlösungen behandelt und in wechselnder Folge in die Lösung eines Polykations, eines Polyanions und eines Detergens (beispielsweise Natriumdodecylsulfat) getaucht, wobei die Kapselhülle jeweils durch Adsorption des Polymeren aus der Lösung an der Kapselaußenfläche und durch Vernetzen der adsorbierten Polymereinzelschichten schrittweise aufgebaut wird.

Diese Prozedur wird erfindungsgemäß mehrfach mit verschiedenen Konzentrationen und/oder chemischen Zusammensetzungen der Polyanion- und Polykationlösungen wiederholt, um die gewünschten Eigenschaften, wie z.B. Dicke und Maschenweite der Einzelschichten zu erzielen.

Außerdem ist empfohlen, die Kügelchen im Korb in Abhängigkeit vom jeweiligen Kernmaterial und vor dem Aufbringen der Einzelschichten, zunächst in geeignete Lösungen zu tauchen, um sie zu härten. Bekannt und gut geeignet sind Lösungen, die Calzium-, Strontium- oder Barium-Ionen enthalten.

Die fertigen Mikrokapseln werden mit verdünntem Natriumalginat behandelt, um ein Verkleben während der Lagerung zu verhindern, und abschließend bis zur Verwendung in Wasser, Pufferlösung oder Kultursubstrat gelagert.

Diese Grundprozedur kann im speziellen Einzelfall variiert werden. So kann zweckmäßig sein, die Polyanionen und Polykationen durch neutrale Polymere teilweise oder ganz zu ersetzen oder mit ihnen zu mischen. Es kann vorteilhaft sein, die Einzelschichten oder die fertigen Mikrokapseln durch Nachbehandlung mit geeigneten di- oder poly-funktionellen Reagenzien zusätzlich zu vernetzen und es kann sich empfehlen, die abschließende Deckschicht zu modifizieren, um die Verträglichkeit mit dem Gewebe eines Wirtsorganismus zu verbessern.

Eine Vorrichtung zur Herstellung kleiner Tröpfchen oder Kügelchen zeichnet sich durch eine im Volumen veränderbare Kompressionskammer aus. Diese ist mit magnetostriktivem oder piezoelektrischem Material verbunden, das sich aufgrund eines von einem Elektromagneten erzeugten Magnetfeldes oder von am Piezomaterial angebrachten Elektroden ausgehenden elektrischen Feldes dehnt oder zusammenzieht und dabei eine Volumenänderung der Kompressionskammer erzeugt. Kernmaterial wird durch eine Zuführungsleitung vom Vorratsbehälter in die Kompressionskammer geführt; in dieser Zuführungsleitung ist ein Rückschlagventil angeordnet, das schließt, sobald der Druck in der Kompressionskammer größer als der Druck im Vorratsbehälter ist und somit einen Druckaufbau in der Kompressionskammer ermöglicht. Nach dem vollständigen Füllen der Kompressionskammer mit Kernmaterial und einer periodisch angeregten Volumenänderung dieser, entstehen durch die Druckänderung an einer Düsenöffnung kleine Tröpfchen von sehr genau reproduzierbar definiertem Volumen, deren Durchmesser im Bereich von etwa 0,3 bis 3 mm gezielt eingestellt werden kann. Die Gestaltung der periodische Strom- oder Spannungsimpulse liefernden Quelle ist nach den Regeln moderner Schaltungstechnik ohne weiteres möglich und bedarf daher keiner näheren Beschreibung. Das Volumen der Tröpfchen ist zur Amplitude der Impulse proportional.

Zur Unterstützung der Tröpfchenbildung ist es zweckmäßig, die Düsenöffnung in axialer Richtung von einem Gasstrom umfließen zu lassen, der zur Erhöhung der Reproduzierbarkeit durch ein Präzisionsventil steuerbar und durch ein Venturirohr und einen Flußmesser kontrollierbar ist.

Die so gebildeten Tröpfchen fallen in eine geeignete Salzlösung, in der sie zu Gelkügelchen erhärten.

Weitere Einzelheiten, Merkmale und Vorteile der Erfindung lassen sich dem nachfolgenden Beschreibungsteil entnehmen, in dem anhand der Zeichnungen Ausführungsbeispiele der Erfindung näher erläutert werden. Sie zeigen in schematischer Darstellung in
- Figur 1: eine Vorrichtung zur Erzeugung kleiner Tröpfchen unter Ausnützung des Magnetostriktionseffektes
- Figur 2: eine Vorrichtung entsprechend Figur 1 unter Ausnützung des piezoelektrischen Effektes
- Figur 3: die Auffangvorrichtung für die erzeugten Tröpfchen mit dem Härtungsbad und dem feinmaschigen Korb zur weiteren Behandlung der Kügelchen.

Die Vorrichtung besteht in in ihrem grundsätzlichen Aufbau aus einem Vorratsbehälter für Kernmaterial (7), einer Zuführungsleitung (2) und einer mit einer Düse (8) versehenen Kompressionskammer (1). Das Kernmaterial wird durch einen Überdruck im Vorratsbehälter (7) mittels der Zuführungsleitung (2) in die Kompressionskammer (1) transportiert. Zur Ermöglichung eines Druckaufbaus in der Kompressionskammer ist die Kernmaterial-Zuführungsleitung (2) mit einem in den Zeichnungen nicht dargestellten Rückschlag-Ventil versehen.

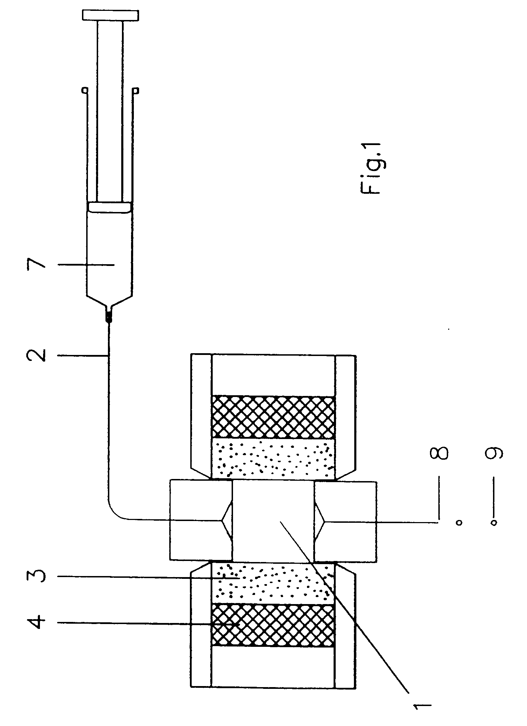

In Figur 1 ist ersichtlich, daß die Kompressionskammer (1) von einem Ring aus einer Magnetostriktions-Legierung (3) und einem Elektromagneten (4) umgeben ist. Bei Stromfluß durch den Elektromagneten (4) verändert die Magnetostriktions-Legierung (3) aufgrund des Magnetfeldes ihre Abmessungen, wodurch sich das Volumen der Kompressionskammer ändert und ein Druckaufbau stattfindet. Dadurch wird Kernmaterial durch die Düse (8) gepreßt und es entstehen kleine Tröpfchen (9), die nach dem erfindungsgemäßen Verfahren weiter verarbeitet werden.

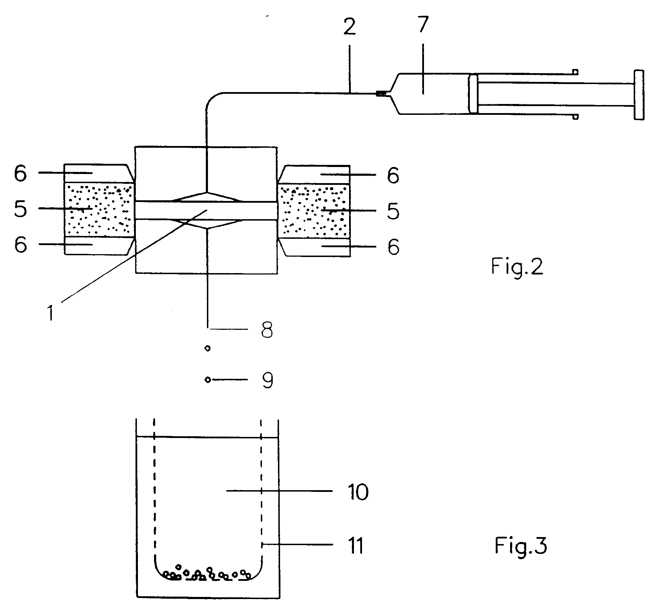

Die in Figur 2 dargestellte Vorrichtung arbeitet mit piezoelektrischem Material (5), das mit Elektroden (6) und einer Spannungsquelle verbunden ist. Ein Anlegen eines Spannungsimpulses an die Elektroden verändert hier ebenfalls das Volumen der Kompressionskammer (1), führt zu einem Druckaufbau und zu an der Düse (8) entstehenden kleinen Tröpfchen (9), die besonders gut größen-reproduzierbar sind. Die relative Volumenänderung der Kompressionskammer ist in beiden dargestellten Ausführungsformen wegen des gewünschten kleinen Kugelvolumens nur gering.

In Figur 3 ist ersichtlich, wie die mit Hilfe einer der beschriebenen Vorrichtungen erzeugten Tröpfchen aus Kernmaterial (9), in eine Lösung (10) fallen, in der sie zu Kügelchen erstarren. Die Anordnung ist so gestaltet, daß diese Kügelchen sich in einem feinmaschigen Korb (11) sammeln, in dem sie auf die erfindungsgemäße Art und Weise weiter behandelt werden.

## Patentansprüche

1. Mikrokapsel, insbesondere zum Einsetzen in Gewebe von Lebewesen oder für biotechnologische Anwendungen, mit einem lebende Zellen und/oder Enzyme enthaltenden, vorzugsweise kugelförmigen Kern und einer ihn umschließenden Hülle, wobei die Hülle in radialer Richtung aus mehreren übereinander angeordneten Einzelschichten aufgebaut ist und die Einzelschichten den Kern allseitig vollständig umschließen, **dadurch gekennzeichnet,** daß
die Einzelschicht aus einem Netzwerk miteinander verflochtener Makromoleküle besteht, die eine poröse Membran bilden,
daß die Poren der Einzelschichten in radialer Richtung zur Bildung eines Durchlasses miteinander in Verbindung stehen,
daß die benachbarten Einzelschichten kovalent und/oder elektrostatisch aneinander gebunden sind,
daß mindestens eine der Einzelschichten mechanisch stabil aufgebaut ist,
und daß mindestens eine der Einzelschichten eine Maschenweite aufweist, die größer oder gleich dem Durchmesser der größten, zur Versorgung der im Kern enthaltenen Zellen benötigten und/oder der den Enzymen zugeführten und/oder von ihnen produzierten Moleküle ist.

2. Mikrokapsel nach Anspruch 1, **dadurch gekennzeichnet,** daß die innere Einzelschicht mit dem Kern biologisch verträglich ist.

3. Mikrokapsel nach Anspruch 1 oder 2, **dadurch gekennzeichnet,** daß die äußere Einzelschicht mit dem Gewebe und/oder der Umgebung der Mikrokapsel biologisch verträglich ist.

4. Mikrokapsel nach einem der Ansprüche 1 bis 3, **dadurch gekennzeichnet,** daß eine Einzelschicht aus einem Stoff mit ionischer und/oder kovalenter Bindung aufgebaut ist.

5. Mikrokapsel nach einem der Ansprüche 1 bis 4, **dadurch gekennzeichnet,** daß eine Einzelschicht aus einem Polymer aufgebaut ist.

6. Mikrokapsel nach einem der Ansprüche 1 bis 5, **dadurch gekennzeichnet,** daß eine Einzelschicht aus einem anionischen und/oder aus einem kationischen und/oder aus einem neutralen Polymer und/oder aus einem Polyanion-Polykation-Polymer (Polyampholyt) aufgebaut ist.

7. Mikrokapsel nach einem der Ansprüche 1 bis 6, **dadurch gekennzeichnet,** daß das Polyanion Polyacrylsäure und/oder Polymethacrylsäure und/oder Polyvinylsulfonsäure und/oder Polyvinylphosphonsäure und/oder Schwefelsäureester polymerer Kohlenhydrate ist.

8. Mikrokapsel nach einem der Ansprüche 1 bis 7, **dadurch gekennzeichnet,** daß das Polykation Polyethylenimin und/oder Polydimethyldiallylammonium und/oder Chitosan ist.

9. Verfahren zur Herstellung einer Mikrokapsel nach einem der Ansprüche 1 bis 8, **dadurch gekennzeichnet,** daß Kügelchen oder andersgeformte Partikel aus dem Kernmaterial durch Rühren und Bewegen in Schwebe gehalten werden,
daß diese Kügelchen oder Partikel nacheinander in Wasser oder eine Salzlösung, in die Lösung eines Polymers und in die Lösung eines Detergens, beispielsweise Natriumdodecylsulfat getaucht werden
und daß diese Prozedur bei verschiedenen Konzentrationen und/oder chemischen Zusammensetzungen von Polymere enthaltenden Lösungen wiederholt wird.

10. Verfahren nach Anspruch 9, **dadurch gekennzeichnet,** daß die Kügelchen oder Partikel zunächst in sie härtende, bevorzugt Calzium- und/oder Strontium- und/oder Barium-Ionen enthaltende Lösungen getaucht werden,
daß die Kügelchen oder Partikel (9) nach dem Aufbringen der Einzelschichten in verdünntes Natriumalginat getaucht werden,
und daß die Kügelchen (9) abschließend in Wasser oder in einer Pufferlösung oder in einem Kultursubstrat gelagert werden.

11. Verfahren nach einem der Ansprüche 9 oder 10, **dadurch gekennzeichnet,** daß die Kügelchen oder Partikel vor den Tauchvorgängen in einen feinmaschigen Korb eingeführt werden.

12. Vorrichtung zur Herstellung von Tröpfchen und Kügelchen zur Verwendung in einem der Verfahren nach Anspruch 9 bis 11, **dadurch gekennzeichnet,** daß eine Kompressionskammer (1) mit einer Zuführungsleitung und einer Düse (8) verbunden ist,
daß zur Volumenänderung der Kompressionskammer (1) magnetostriktives Material (3) mit einem daran angeordneten Elektromagneten (4) oder piezoelektrisches Material (5) mit daran angeordneten Elektroden (6) verwendet ist, daß in der zu einem Vorratsbehälter (7) für Kernmaterial führenden Zuführungsleitung (2) ein Rückschlag-Ventil angeordnet ist und
daß der Elektromagnet (4) oder die Elektroden (6) mit einer periodische Impulse liefernden Strom- oder Spannungsquelle verbunden sind.

13. Vorrichtung nach Anspruch 12, **gekennzeichnet durch** eine in axialer Richtung gasumströmte Düsenöffnung (8).

## Claims

1. Microcapsule, in particular for insertion into tissue of organisms or for biotechnological applications, having a preferably spherical core which contains living cells and/or enzymes and an enclosing shell, whereby in a radial direction the shell comprises several superimposed single layers and the single layers enclose the core fully on all sides, **wherein**
the single layer consists of a network of interlaced macromolecules which form a porous membrane,
the pores of the single layers are interconnected in a radial direction to form a passage,
the adjacent single layers are bonded covalently and/or electrostatically,
at least one of the single layers is of stable structure,
and at least one of the single layers has an aperture width which is greater than or equal to the diameter of the largest molecule required to supply the cells contained in the core and/or supplied to the enzymes and/or produced by them.

2. Microcapsule according to claim 1, **wherein** the inside single layer is biologically compatible with the core.

3. Microcapsule according to claim 1 or 2, **wherein** the outer single layer is biologically compatible with the tissue and/or the environment of the microcapsule.

4. Microcapsule according to one of claims 1 to 3, **wherein** a single layer is built up from a substance with ionic and/or covalent bonds.

5. Microcapsule according to one of claims 1 to 4, **wherein** one single layer is built up from one polymer.

6. Microcapsule according to one of claims 1 to 5, **wherein** one single layer is built up from an anionic and/or a cationic and/or a neutral polymer and/or from a polyanion-polycation polymer (polyampholyte).

7. Microcapsule according to one of claims 1 to 6, **wherein** the polyanion is polyacrylic acid and/or polymethacrylic acid and/or polyvinylsulphonic acid and/or polyvinylphosphonic acid and/or sulphuric acid esters of polymer carbohydrates.

8. Microcapsule according to one of claims 1 to 7, **wherein** the polycation is polyethylenimine and/or polydimethyldiallyl ammonium and/or chitosan.

9. Method to prepare a microcapsule according to one of claims 1 to 8, **wherein** beads or otherwise formed particles from the core material are kept in suspension by agitation and motion,
these beads or particles are immersed successively into water or a salt solution, into the solution of a polymer and into the solution of a detergent, for example, sodium dodecyl sulphate
and this procedure is repeated for different concentrations and/or chemical compositions of solutions containing polymers.

10. Method according to claim 9, **wherein** the beads or particles are initially immersed into solutions that harden them containing preferably calcium and/or strontium and/or barium ions,
the beads or particles (9) are immersed into diluted sodium alginate after the application of the single layers,
and the beads (8) are finally stored in water or a buffer solution or in a culture substrate.

11. Method according to one of claims 9 or 10, **wherein** the beads or particles are introduced into a fine-meshed basket prior to the immersion processes.

12. Device for the preparation of droplets and beads for use in one of the methods according to claims 9 to 11, **wherein** a compression chamber (1) is connected to supply line and a nozzle (8),
to change the volume of the compression chamber (1) magnetostrictive material (3) with an electromagnet (4) disposed thereupon or piezoelectric material (5) with electrodes (6) disposed thereupon is used,
a return valve is disposed in the supply line (2) leading to a supply container (7) for core material and
the electromagnet (4) or the electrodes (6) are connected to a power or voltage source that supplies a periodic pulse.

13. Device according to claim 12, **wherein** there is a nozzle opening (8) around which flows gas in an axial direction.

## Revendications

1. Microcapsule, notamment destinée à l'implantation d'organismes dans les tissus ou aux applications biologiques, comprenant un noyau de préférence de forme sphérique, entouré d'une gaine, cette dernière étant composée de plusieurs couches superposées dans le sens radial, ces différentes couches enveloppant entièrement le noyau, **caractérisée en ce que**
chaque couche est composée d'un réseau de macromolécules entrelacées, formant une membrane poreuse,
les pores des différentes couches sont reliés entre eux dans le sens radial et forment un passage,
les couches avoisinantes sont reliées par une liaison covalente et/ou électrostatique,
une couche au moins possède une structure mécanique stable,
et en ce que l'ouverture de malle d'une couche au moins est supérieure ou égale au diamètre de la plus grosse molécule nécessaire à l'alimentation des cellules enfermées dans le noyau et/ou de la molécule alimentant les enzymes et/ou produite par ces dernières.

2. Microcapsule selon la revendication 1, **caractérisée en ce que la** couche intérieure est biologiquement compatible avec le noyau.

3. Microcapsule selon la revendication 1 ou 2, **caractérisée en ce que** la couche extérieure est biologiquement compatible avec le tissu et/ou l'environnement de la microcapsule.

4. Microcapsule selon l'une des revendications 1 à 3, **caractérisée en ce que** l'une des couches est composée d'une substance à liaison ionique et/ou covalente.

5. Microcapsule selon l'une des revendications 1 à 4**, caractérisée en ce que** l'une des couches se compose d'un polymère.

6. Microcapsule selon l'une des revendications 1 à 5, **caractérisée en ce que** l'une des couches se compose d'un polymère anionique et/ou d'un polymère cationique et/ou d'un polymère neutre et/ou d'un polymère polyanion-polycation (polyampholyte).

7. Microcapsule selon l'une des revendications 1 à 6, **caractérisée en ce que** le polyanion est un acide polyacrylique et/ou un acide polyméthacrylique et/ou un acide polyéthylène sulfonique ou polyvinyle-phosphone et/ou un ester sulfurique de carbohydrates polymériques.

8. Microcapsule selon l'une des revendications 1 à 7, **caractérisée en ce que** le polycation est une polyéthyléne-imine et/ou un ammonium de polydiméthyle-dially et/ou du chitosan.

9. Procédé de fabrication d'une microcapsule selon l'une des revendications 1 à 8, **caractérisé en ce que** les petites boules ou les particules de matière du noyau d'une autre forme sont maintenues en suspension et en mouvement par agitation,
et en ce que l'on plonge ces petites boules successivement dans de l'eau ou dans une solution saine, dans une solution de polymère et dans une solution de détergent, comme par exemple du dodécylsulfate de sodium,
et en ce que l'on répète cette procédure avec des solutions de polymères de concentration et/ou de composition chimique différentes.

10. Procédé selon la revendication 9, **caractérisé en ce que** l'on trempe d'abord les petites boules ou les particules (9) dans des solutions durcissantes, contenant de préférence des ions calcium et/ou strontium et/ou baryum,
et en ce que les petites boules (9) tombent finalement dans de l'eau ou dans une solution tampon ou dans un substrat de culture.

11. Procédé selon l'une des revendications 9 ou 10, **caractérisé en ce que** l'on place les petites boules ou les particules dans un panier à malles fines avant de les plonger dans les solutions.

12. Dispositif de génération de gouttelettes et de petites boules en vue de l'utilisation dans l'un des procédés selon les revendications 9 à 11, **caractérisé en ce qu'une** chambre de compression (1) est reliée à un conduit d'alimentation et à une buse (8),
et en ce que l'on utilise un matériau magnétostrictif (3) et un électro-aimant (4) ou un matériau piézo-électrique (5) relié à des électrodes (6) pour faire varier le volume de la chambre de compression (1),
et en ce que le conduit d'alimentation (2) transportant la matière du noyau entre le réservoir (7) et la chambre de compression, renferme un clapet anti-retour,
et en ce que l'électro-aimant (4) ou les électrodes (6) sont reliés à une source de courant ou de tension délivrant des impulsions périodiques.

13. Dispositif selon la revendication 12, **caractérisé en ce qu'un** courant de gaz circule dans le sens axial autour de l'orifice de la buse (8).
